# EUROPEAN PATENT SPECIFICATION

(11) **EP 4 190 365 B1**
(45) Date of publication and mention of the grant of the patent: **20.08.2025**
(21) Application number: 22210174.3
(22) Date of filing: 29.11.2022
(51) Int. Cl.: A61L 2/07, A61L 2/24, A61L 2/26, B08B 3/00

(54) **AUTOCLAVE**
AUTOKLAV
AUTOCLAVE

(30) Priority: 06.12.2021 IT 202100030749; 25.11.2022 IT 202200024285
(43) Date of publication of application: 07.06.2023
(73) Proprietor: CEFLA SOCIETA' COOPERATIVA, 40026 Imola (BO) (IT)
(72) Inventor: Vergara, Gabriele, 40137 Bologna BO (IT); Spettoli, Paolo, 40026 Imola BO (IT); Ragazzini, Luca, 40026 Imola BO (IT)
(74) Representative: Karaghiosoff, Giorgio Alessandro

(56) References cited:
- EP-A1- 1 273 311
- EP-B1- 1 273 311
- CN-A- 112 316 162
- CN-A- 115 068 640
- US-A- 4 909 988

## Description

The present invention relates to the technical field of autoclaves for sterilizing surgical/dental/veterinary instruments. More particularly, the invention relates to a hydraulic circuit comprising an ejector for suctioning the content of the sterilization chamber.

It hardly needs be mentioned that sterilizing means performing a physical or chemical process destroying all microorganisms, including bacterial spores.

In the art, there are provided substantially two types of autoclaves:
- Autoclaves working with water steam;
- Autoclaves working with chemical vapours, e.g. alcohol, formaldehyde, ketones, ethylene oxide, etc. and/or mixtures thereof.

The present invention can be used in bench-top autoclaves, or small autoclaves, working with water steam, which are commonly found in medical/dental/veterinary practices making use of re-usable instruments needing sterilization after their use.

Water steam autoclaves are supplied with water, usually demineralized water, which is brought to high temperature and pressure. The water steam so generated is conveyed inside a sterilization chamber, wherein the instruments to be sterilized are placed, often inside sterilization pouches or suitable sterilization cassettes. The contact at a known pressure and for a pre-set time between water steam and instruments allows to sterilize said instruments.

A sterilization cycle comprises many steps:
- Manual loading of the autoclave sterilization chamber with the material to be sterilized, suitably placed inside sterilization pouches/cassettes;
- Pre-heating of the sterilization chamber and of the load;
- Emptying of the sterilization chamber, in order to remove atmospheric air; said emptying can occur with a plurality of successive steps of pressurization and emptying;
- Pressurization of the sterilization chamber, wherein the sterilization chamber is filled with water steam at high temperature (about 121°-135°C) and high pressure (about 1.10 and 2.10 bar);
- Actual sterilizing, through the contact between high temperature and pressure water steam and objects to be sterilized for a pre-set time;
- Removing water steam from the sterilization chamber;
- Drying and pressure balancing;
- Manual removal of the sterilized objects from the sterilization chamber.

In the art, it is known to perform said pre-heating step of the sterilization and of the load to be sterilized by using suitable heating means (e.g. an electrical resistance) applied to said sterilization chamber.

The drying step typically comprises maintaining a suitable temperature in the sterilization chamber and of the sterilized load, maintaining a negative pressure inside the sterilization chamber (i.e. a pressure lower than the atmospheric pressure), and optionally generating an airflow at atmospheric pressure that reaches the sterilization chamber passing through a bacteriologic filter.

Typically, in an autoclave there are provided at least two kinds of sterilization cycles:
- A more rapid cycle wherein the objects to be sterilized are kept in contact with water steam at a temperature of 135°C and a pressure of 2.10 for at least 3,5 minutes;
- A more delicate cycle, wherein the objects to be sterilized are kept in contact with water steam at a temperature of 121°C and a pressure of di 1.10 bar for at least 20 minutes.

In the art, there are provided class B, class S, class N autoclaves.
- The autoclaves suitable for performing class B (Big small sterilizers) cycles according to EN ISO 13060 standard are provided with an embedded vacuum pump and can perform fractionated pre-vacuum air removal. Shortly, before starting the sterilizing step, the autoclave performs a sequence of emptying and filling steps of the sterilization chamber with water steam. In this way, the autoclave can remove air bubbles even from the small cavities or porosities of instruments, allowing steam to enter into contact with the internal and external surfaces of instruments, so as to sterilize them perfectly. With class B autoclaves, all kinds of materials can be sterilized: porous materials, pouched materials, textiles, and hollow bodies like handpieces, turbines and cannulas.
- EN ISO 13060 standard defines the features of the autoclaves performing class S cycles. Such autoclaves are generally provided with a vacuum pump, and can or cannot perform fractionated pre-vacuum air removal. They ensure the sterilization of loads as specified by the autoclave manufacturer.
- The autoclaves performing class N cycles do not perform fractionated pre-vacuum air removal, are usually not provided with a vacuum pump, and do not dry the objects. They can be used just for solid materials like e.g. a not-pouched dental mirror.

The autoclave according to the present invention is preferably an autoclave capable of performing class B sterilization cycles.

In the known art, typically autoclaves comprise the following components:
- a couple of water tanks: a first tank for demineralized water that is poured by a human operator in order to fill it, a second tank for used water, deriving from steam condensation; according to autoclave models, said used water can be conveyed to the mains drain, or recycled, preferably undergoing a previous filtration step, for successive sterilization cycles;
- a pump to convey water toward
- a steam generator, whereto water is injected in order to produce steam; from here, steam is conveyed to
- a sterilization chamber; from here, steam is conveyed to
- a condenser, wherein steam is condensed and from which water, reverted to its liquid state, is conveyed toward the used water tank;
- a plurality of solenoid valves and tubing allowing to suitably connect the above-mentioned components.

Providing autoclaves with a system allowing to extract both air and water steam from the sterilization chamber is known in the art, e.g. in the two documents of the known art in the following.

EP0992247A1 of Dentalwerk Buermoos GmbH describes an autoclave provided with a vacuum pump extracting water steam from the sterilization chamber. To this aim, a vacuum pump, preferably a membrane pump, is used to remove steam from the sterilization chamber, in conjunction with a condenser which is intended to bring the steam to a pressure and temperature wherein steam goes back to liquid state. It is worth mentioning that the vacuum pump is probably one of the most expensive components of an autoclave.

EP3342429A1 of W & H Sterilization describes a refinement of the preceding invention, wherein the sterilization chamber is connected with two three-way valves allowing to inject a pre-set quantity of hot steam directly inside the condenser; said steam is cooled and condensed inside said condenser, and therefore its volume and pressure decrease to levels lower than those of the sterilization chamber. Said condenser is placed in direct communication with the sterilization chamber in order to perform steam removal. CN112316162A and CN115068640 of Shandong Shinva Med Instr CO describe instead a hydraulic circuit for an autoclave comprising a boiler that can be considered like the combination of a reservoir of water used to generate steam and of an element generating steam, a pump, a sterilization chamber, two steam condensers, a manifold. Although not explicitly named as such, said hydraulic circuit of the autoclave comprise a Venturi tube that works through steam (paragraph 0037: In the steam ejector 4, the steam is accelerated and sprayed from the steam inlet to the steam outlet. When the high-speed steam passes through the evacuation port between the steam inlet and the steam outlet, a negative pressure is generated at the outlet of the evacuation port, and this negative pressure can realize the extraction. The air and moisture in the sterilization chamber 11 realize the vacuuming of the sterilization chamber 11).

Aim of the present invention is providing an autoclave allowing to remove air/water steam from the sterilization chamber without a vacuum pump, so that a cheaper autoclave is obtained by removing one of its costlier components.

This object is achieved by an apparatus and a method having the features of the independent claims. Advantageous embodiment and refinements are specified in the claims dependent thereon.

Substantially, the solution consists in replacing the vacuum pump with an ejector, which, exploiting the Venturi effect, allows to generate a suction flow capable of removing residual air/water steam from the sterilization chamber.

The ejector is a known commercial component, used for a variety of other applications.

In a first embodiment, the ejector is actuated by a compressed air flow coming from outside the autoclave, e.g. from the compressed air circuit customarily provided in a dental practice.

In a second embodiment, the ejector is actuated by the water steam flow generated by the autoclave itself.

In a third, preferred embodiment, said Venturi ejector works using steam that is produced starting by the condensed water coming back from the sterilization chamber of the autoclave. The steam used to actuate the ejector (Venturi tube) is further condensed and conveyed to the used water tank, so that used water is used many times.

In an embodiment, the ejector is placed downstream the steam condense. and in discharge, upstream the manifold.

In an alternative embodiment, the ejector is placed immediately downstream a manifold and upstream the condenser.

The advantages according to the present invention as defined in claims 1 and 7 are manifold.

A first advantage is replacing a costly component (the vacuum pump) with a cheaper component (the ejector). In a preferred embodiment, the cost of the autoclave is further reduced by using a plastic ejector, less expensive than the same component made of metal.

A second advantage lies in the fact that the ejector is a static component, and therefore suffers less from wear with respect to a vacuum pump. Therefore, downtimes risk is reduced.

A third advantage is placing said ejector immediately downstream the solenoid valves group (manifold) but upstream the steam condenser: in this way, the suctioning action performed by the ejector is even stronger.

A fourth advantage is generating steam with used water, that is water obtained from the condensation of the steam used in the sterilization chamber, which came into contact with contaminated instruments. In this way, the demineralized water is used for two distinct operations: a first time to generate the steam intended for sterilization; and once it ended up in the user water tank, many times in order to generate the steam intended for actuating the ejector. In this way the consumption of demineralized water is greatly reduced.

Further advantages and properties of the present invention are disclosed in the following description, in which exemplary embodiments of the present invention are explained in detail based on the drawings:
Figure 1 Axonometric view of a bench-top autoclave;
Figure 2 Schematic representation of the autoclave hydraulic circuit according to a first example which is not part of the invention;
Figure 3 Schematic representation of the autoclave hydraulic circuit according to a second example which is not part of the invention;
Figure 4 Schematic representation of the autoclave hydraulic circuit according to the invention;
Figure 5 Schematic representation of an ejector.

It is worth mentioning that the solenoid valves shown in the drawings and mentioned in the Description use the following abbreviations: NC means normally closed, NA means normally open, while 3V means three-way.

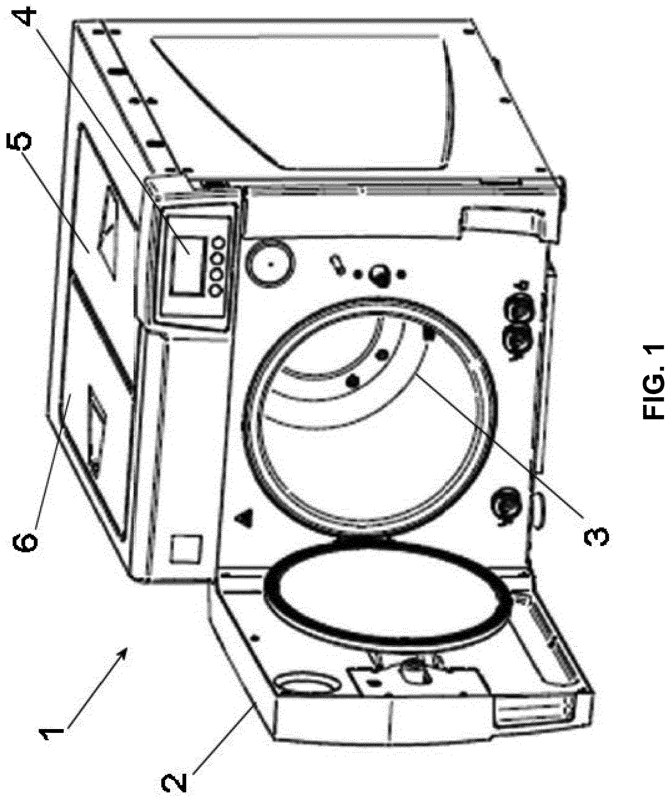

Figure 1 shows an axonometric view of a typical bench-top autoclave 1. The autoclave 1 comprises a sterilization chamber 3 that is hermetically closed by a door 2. A display 4 placed on its front allows a human operator to input the different sterilization cycles and to receive information about the status of the autoclave 1. Above the sterilization chamber 3 there are provided two water tanks: a first tank 5 is provided for demineralized water, while a second tank 6 is provided for water condensed from steam, i.e. already used water.

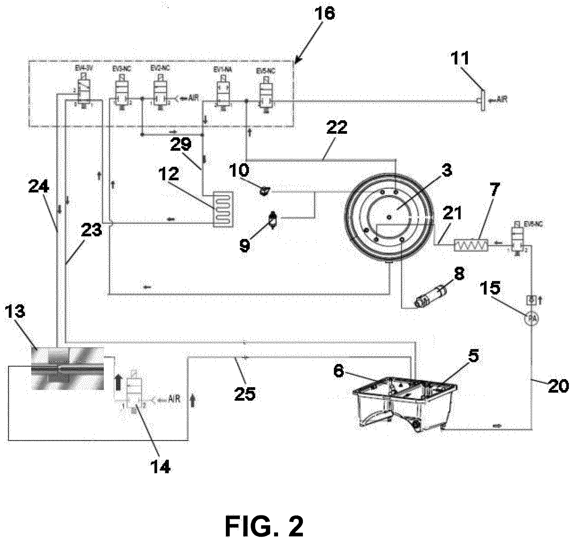

Figure 2 shows a schematic representation of an autoclave according to a first example which is not part of the invention, wherein said ejector is actuated by compressed air.

The hydraulic circuit according to the first embodiment comprises:
- two water tanks 5, 6;
- a pulse pump 15 for injecting water into a steam generator;
- a two-way, normally closed solenoid valve EV6-NC;
- a steam generator 7;
- a sterilization chamber 3;
- a safety valve 8;
- a pressure transducer 9;
- a pressure switch 10;
- a bacteriologic air filter 11;
- a manifold 16 (solenoid valve group) comprising five solenoid valves;
- a condenser 12;
- an ejector 13;
- a two-way, normally closed solenoid valve EV8-NC 14 connected to the compressed air net.

The demineralized water contained in the clear water tank 5 is conveyed through a tubing 20 firstly to the pulse pump 15, successively to a solenoid valve EV6-NC, and to the steam generator 7 inside which it is transformed into steam. Steam is conveyed through a tubing 21 to the sterilization chamber 3.

Connected to the sterilization chamber there are provided the safety valve 8 for preventing overpressures, the pressure transducer 9 to detect the pressure inside said sterilization chamber 3, the pressure switch 10 that does not allow the opening of the door 2 when the pressure inside the sterilization chamber is higher than atmospheric pressure.

Through a tubing 22, the manifold 16 is connected to said sterilization chamber 3. Said manifold 16 comprises five solenoid valves. A first EV5-NC solenoid valve controls the airflow incoming through said bacteriologic filter 11 in the pressure-balancing step at the end of sterilization cycles. A second solenoid valve EV1-NA connected to the top portion of the sterilization chamber 3 allows to control steam discharge. A third solenoid valve EV2-NC connected to atmospheric air allows to control an air flow helping to empty tubings. A fourth solenoid valve EV3-NC connected to the bottom portion of the sterilization chamber 3 allows to control the discharge of the condensed from steam. The two solenoid valves EV1-NA and EV3-NC convey the flow of steam/condense to the condenser 12 through a tubing 29. A fifth solenoid valve EV4-3V connected to the condenser 12 allows to convey the flow of steam/condense alternatively:
- toward the used water tank 6 through a tubing 23, when the solenoid valve in its off condition, or
- toward the ejector 13 through a tubing 24, when the solenoid valve is in its on condition.

The water/condense outcoming from the ejector 13 is finally conveyed to the used water tank 6 through a tubing 25.

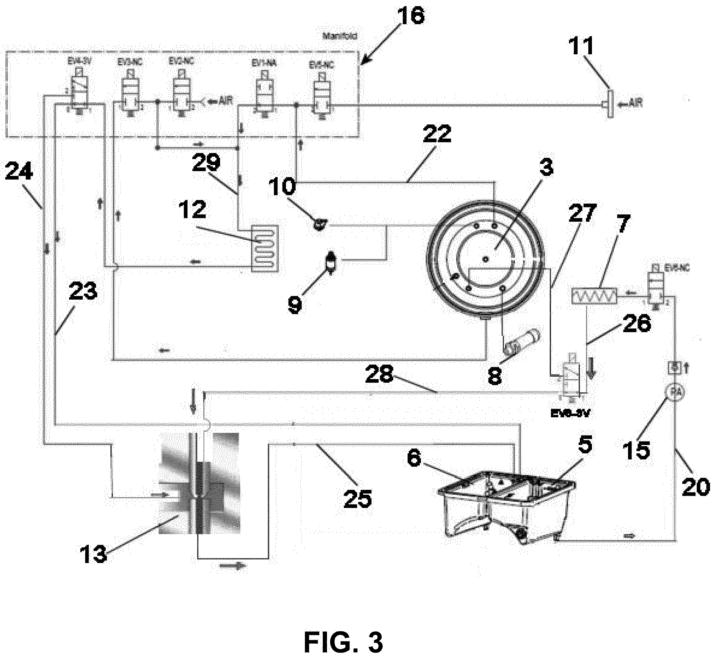

Figure 3 shows a schematic representation of the hydraulic circuit of an autoclave according to a second example which is not part of the invention, wherein the ejector is actuated by a steam flow generated by the steam generator 7 of the autoclave 1.

The hydraulic circuit according to the second embodiment comprises:
- two water tanks 5, 6;
- a pulse pump 15 for injecting water into a steam generator;
- a first two-way, normally closed solenoid valve EV6-NC;
- a steam generator 7;
- a second solenoid valve EV8-3V allowing to convey the steam flow alternatively toward the sterilization chamber 3 or to the ejector 13;
- a sterilization chamber 3;
- a safety valve 8;
- a pressure transducer 9;
- a pressure switch 10;
- a bacteriologic air filter 11;
- a manifold 16 (solenoid valve group) comprising five solenoid valves;
- a condenser 12;
- an ejector 13.

The demineralized water contained in the clear water tank 5 is conveyed through a tubing 20 firstly to the pulse pump 15, successively to a solenoid valve EV6-NC, and to the steam generator 7 inside which it is transformed into steam. Steam is conveyed through a tubing 26 to the valve EV8-3V, which according to its state conveys it alternatively to the sterilization chamber 3 through a tubing 27 or to the ejector 13 through a tubing 28.

Connected to the sterilization chamber there are provided the safety valve 8 for preventing overpressures, the pressure transducer 9 to detect the pressure inside said sterilization chamber 3, the pressure switch 10 that does not allow the opening of the door 2 when the pressure inside the sterilization chamber is higher than atmospheric pressure.

Through a tubing 22, the manifold 16 is connected to said sterilization chamber. Said manifold 16 comprises five solenoid valves. A first EV5-NC solenoid valve controls the airflow incoming through said bacteriologic filter 11 in the pressure-balancing step at the end of sterilization cycles. A second solenoid valve EV1-NA connected to the top portion of the sterilization chamber 3 allows to control steam discharge. A third solenoid valve EV2-NC connected to atmospheric air allows to control an air flow helping to empty tubings. A fourth solenoid valve EV3-NC connected to the bottom portion of the sterilization chamber 3 allows to control the discharge of the condense from steam. The two solenoid valves EV1-NA and EV3-NC convey the flow of steam/condense to the condenser 12 through a tubing 29. A fifth solenoid valve EV4-3V connected to the condenser 12 allows to convey the flow of steam/condense alternatively:
- toward the used water tank 6 through a tubing 23, when the solenoid valve in its off condition, or
- toward the ejector 13 through a tubing 24, when the solenoid valve is in its on condition.

The water/condense outcoming from the ejector 13 is finally conveyed to the used water tank 6 through a tubing 25.

In the first example, the operating principle of the ejector 13 provides that an airflow coming from outside the autoclave, controlled by the solenoid valve EV8-NC 14, is conveyed to the ingress of the ejector, accelerated through its nozzle, so as to generate suction through a Venturi effect. This allows the removal of steam/condense from the sterilization chamber 3, up to pressure values lower than atmospheric pressure (conventionally indicated as 0).

In the second example, the operating principle of the ejector 13 provides that a steam flow generated by the steam generator 7 and controlled by the solenoid valve EV8-3V is conveyed to the ingress of the ejector, accelerated through its nozzle, so as to generate suction through a Venturi effect. This allows the removal of steam/condense from the sterilization chamber 3, up to pressure values lower than atmospheric pressure (conventionally indicated as 0).

In the examples shown in Figures 2 and 3, the ejector 13 is placed downstream the steam condenser 12.

In alternative, not shown embodiments, the ejector 13 is placed immediately downstream the manifold 16 and upstream the condenser 12.

In all the embodiments, the discharge of the condensed steam outcoming from said ejector 13 is conveyed to the used water tank 6 through a tubing 25.

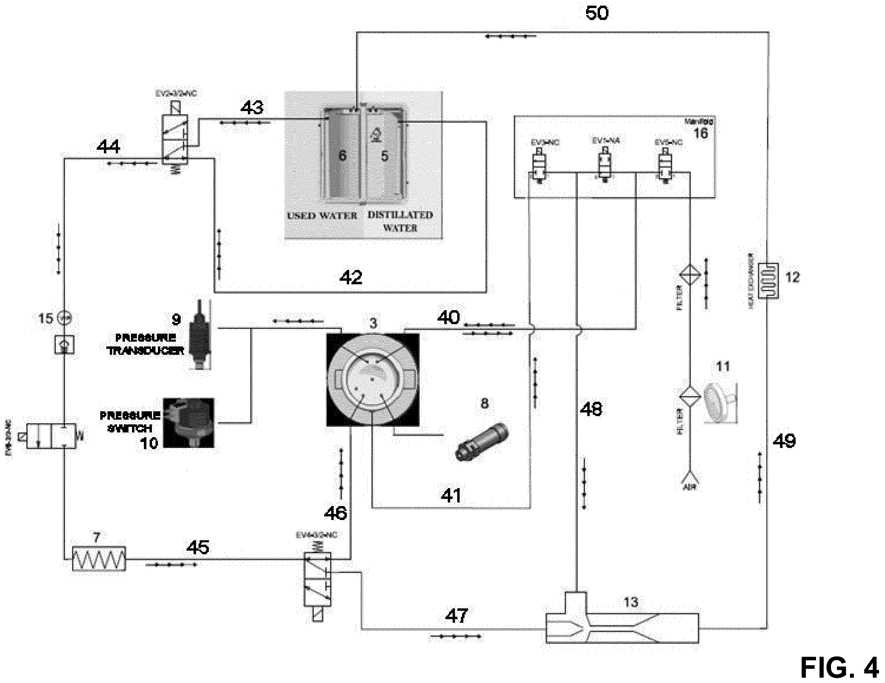

Figure 4 shows a schematic representation of an autoclave as defined in claim 1, wherein an ejector 13 is actuated by a steam flow produced by the steam generator 7 of the autoclave 1 by using used water contained in the discharge tank 6.

The hydraulic circuit comprises:
- a water tank 5 for the demineralized water intended for producing steam for a sterilization chamber 3;
- a water tank 6 for the used water, condensed from the steam coming from the sterilization chamber;
- a pulse pump 15 for injecting water into a steam generator;
- a first two-way, normally closed solenoid valve EV6-NC;
- a steam generator 7;
- a second solenoid valve EV4-3/2-NC allowing to convey the steam flow alternatively toward the sterilization chamber 3 or toward the ejector 13;
- a third solenoid valve EV2-3/2-NC allowing to select the demineralized water tank 5 or the condensed water tank 6 from which water to be conveyed to the circuit can be withdrawn;
- a sterilization chamber 3;
- a safety valve 8;
- a pressure transducer 9;
- a pressure switch 10;
- a bacteriologic air filter 11;
- a manifold 16 (solenoid valve group) comprising three solenoid valves;
- a condenser 12;
- an ejector 13.

The water contained in the tanks 5 and 6 is conveyed trough tubing 42 and 43 first to the solenoid valve EV2-3/2-NC and successively to an impulse pump 15, through a tubing 44. Consecutively, the water is channelled to a solenoid valve EV6-NC and to a steam generator 7 inside which is converted into steam. The steam is channelled through a tubing 45 to the valve EV4-3/2-NC that, according to its actuation state, sends it alternatively to the sterilization chamber 3 through a tubing 46 or to the ejector 13 through a tubing 47.

To the sterilization chamber 3 there are connected the safety valve 8 for preventing overpressures, the pressure transducer 9 for detecting the pressure inside the sterilization chamber, the pressure switch 10 that does not allow the opening of the door 2 when the pressure inside the chamber 3 is higher than the atmospheric pressure.

Through tubing 40 and 41, a manifold 16, comprising three solenoid valves, is connected to the sterilization chamber 3. A first valve EV5-NC controls the input air flow through a bacteriologic filter 11 in the step of pressure levelling at the end of the sterilization cycle. A second solenoid valve EV1-NA connected to the upper portion of the chamber 3 allows to control steam discharge, through the tubing 40. A third solenoid valve EV3-NC connected to the lower portion of the chamber 3 allows to control the discharge of condensed water and steam through the tubing 41. The two solenoid valves EV1-NA e EV3-NC send, through a tubing 48, the flow of steam/condense coming from the chamber 3 to the ejector 13.

The steam generator 7 supplies, through a tubing 47, the ejector 13 allowing the passage, through tubing 48 and 49, of the water/condense coming out from the condenser 12. The water/condense coming out from said condenser 12 is finally sent to the discharge tank 6, through a tubing 50.

The operating principle of the ejector 13 provides that a steam flow generated by the steam generator 7 employing used water withdrawn from the tank 6 through the tubing 43 is controlled by the solenoid valve EV4-3/2-NC. Such steam flow is conveyed to the ingress of the ejector 13, accelerated through its nozzle, so as to generate suction through a Venturi effect. This allows the removal of steam/condense from the sterilization chamber 3, up to pressure values lower than atmospheric pressure (conventionally indicated as 0).

In the embodiment shown in Figure 4, the ejector 13 is placed in suction downstream the manifold 16 and in discharge, upstream the steam condenser 12.

In an alternative (not shown) embodiment, the ejector 13 is placed in suction, downstream the condenser 12, and in discharge, upstream the manifold 6.

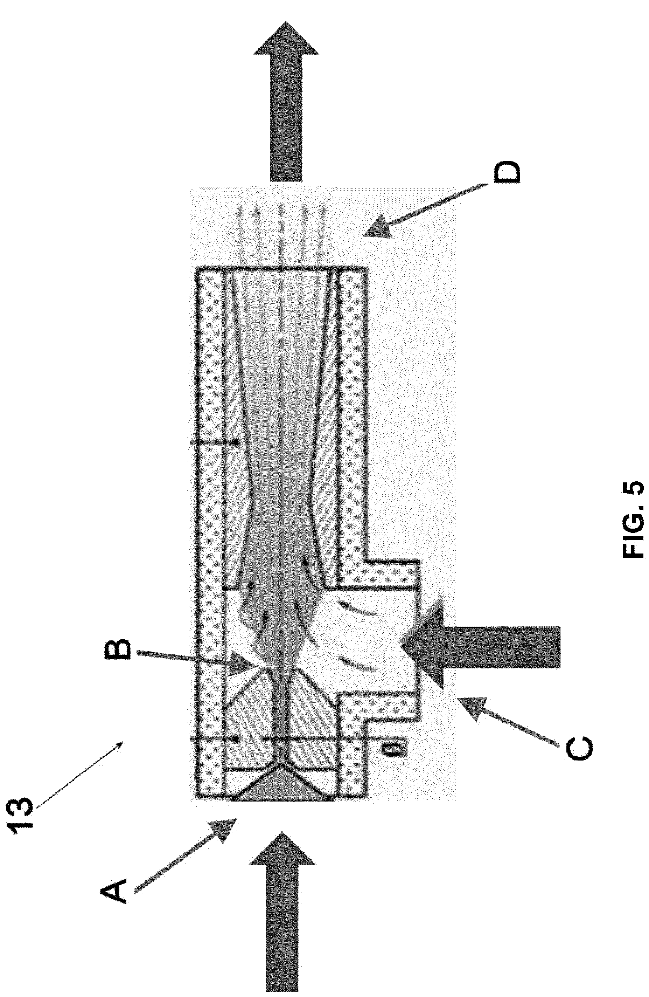

Figure 5 shows the operating principle of a vacuum ejector, i.e. the ejector 13.

Said ejector comprises an ingress A and a nozzle B for the motive fluid, an ingress C for the mixture steam/condense suctioned from the sterilization chamber 3. Downstream said ingresses, perpendicular to each other, there is provided an egress D, in axis with the ingress A and the nozzle B.

In Figure 5, the bold arrows show the ingresses and the egress of the fluids, while the thin arrows show the pathway of said fluids inside the ejector.

The operating principle of the ejector 13, based on the Venturi effect, provides that a pressurized motive fluid (compressed air or steam) is conveyed to the ingress A and accelerated through the nozzle B. At the egress of said nozzle B, the motive fluid flows at an increased speed due to the compression exerted by the nozzle. At the same time the motive fluid generates a suction dragging the steam/condense coming from the sterilization chamber and entering into the ejector 13 through the ingress C. Downstream the ingress C, the motive fluid mixed with the suctioned steam/condense reach the egress D and from there the used water tank 6 through the tubing 25.

In the first example, the motive fluid used is compressed air coming from a compressed air net independent from the autoclave 1.

In the second example, the motive fluid used is steam, generated by the steam generator 7 and conveyed to the ejector 13 through tubing 28.

In the embodiment according to the invention, downstream the ingress C, the motive fluid mixed with the suctioned steam/condense reach the egress D and from there the used water tank 6 through the tubing 49 and 50.

In a preferred embodiment, the ejector 13 is moulded in a suitable technical plastic material, like e.g. PEEK, capable of standing the contact with high-temperature steam. The plastic ejector can cost one tenth the price of the same component made of metal.
- 1: Autoclave
- 2: Door
- 3: Sterilization chamber
- 4: Display
- 5: Clear water tank
- 6: Used water tank
- 7: Steam generator
- 8: Safety valve
- 9: Pressure transducer
- 10: Pressure switch
- 11: Bacteriologic filter
- 12: Heat exchanger
- 13: Ejector
- 14: Solenoid valve
- 15: Pulse pump
- 16: Manifold
- 20: Tubing
- 21: Tubing
- 22: Tubing
- 23: Tubing
- 24: Tubing
- 25: Tubing
- 26: Tubing
- 27: Tubing
- 28: Tubing
- 29: Tubing
- 40: Tubing
- 41: Tubing
- 42: Tubing
- 43: Tubing
- 44: Tubing
- 45: Tubing
- 46: Tubing
- 47: Tubing
- 48: Tubing
- 49: Tubing
- 50: Tubing
- A: Ingress
- B: Nozzle
- C: Ingress
- D: Egress

## Claims

1. Hydraulic circuit for a water steam autoclave (1) for sterilizing instruments, comprising in the order
- a first tank (5) for the demineralized water intended for generating steam for a sterilizing chamber and a second tank (6) for collecting used water,
- a pump (15) for conveying water, in sequence, toward the following units
- a steam generator (7),
- a sterilizing chamber (3) wherein steam and instruments to be sterilized come into contact,
- a steam condenser (12),
- a manifold (16),
which units are connected to each other through suitable tubing (20-29 or 40-50) and solenoid valves,
wherein the hydraulic circuit further comprises an ejector (13) to suction the content of said sterilization chamber (3) and
wherein said ejector (13) is actuated by steam generated from water withdrawn from said second tank (6) for collecting used water,
**characterized in that** the hydraulic circuit comprises a first tubing (43) and a second tubing (42) connecting respectively the said first tank (5) and the said second tank (6) to the said steam generator (7) through a solenoid valve (EV2-3/2-NC) allowing to select the demineralized water tank 5 or the used water tank 6 from which water to be conveyed to the circuit can be withdrawn and a third tubing (45) for channelling the steam to a further valve (EV4-3/2-NC) that according to its actuation state sends it alternatively to the sterilization chamber (3) through a fourth tubing (46) or to the ejector (13) through a fifth tubing (47).

2. Hydraulic circuit for a water steam autoclave (1) for sterilizing instruments according to claim 1, wherein said ejector (13) is placed downstream said manifold (16) and upstream said steam condenser (12).

3. Hydraulic circuit for a water steam autoclave (1) for sterilizing instruments according to claim 1 or 2, wherein said ejector (13) is placed downstream said steam condenser (12) and upstream said used water tank (6).

4. Hydraulic circuit for a water steam autoclave (1) for sterilizing instruments according to claim 2 or 3, wherein the water/condense outcoming from said ejector (13) is conveyed to the used water tank (6) through a tubing (49 and 50), and optionally passing through said condenser (12).

5. Hydraulic circuit for a water steam autoclave (1) for sterilizing instruments according to one or more of the preceding claims, further comprising a safety valve (8), a pressure transducer (9), a pressure switch (10), a bacteriologic air filter (11).

6. Hydraulic circuit for a water steam autoclave (1) for sterilizing instruments according to one or more of the preceding claims, wherein said ejector is made of a suitable technical plastic material, preferably PEEK.

7. Method for performing sterilization cycles using an autoclave (1) comprising the hydraulic circuit according to claims 1 to 6 comprising the following steps:
- Manual loading by a human operator of the sterilization chamber (3) of said autoclave (1) with the material to be sterilized, suitably placed inside sterilization pouches/cassettes;
- Pre-heating of the sterilization chamber (3) and of the load inside said sterilization chamber;
- Emptying of the sterilization chamber (3) to remove the atmospheric pressure air in it; said emptying can optionally occur through successive steps of pressurization and emptying steps;
- Pressurization of the sterilizing chamber (3), wherein the sterilization chamber is filled with high temperature and high-pressure water steam;
- Actual sterilization, through the contact between high temperature and high-pressure steam with the material to be sterilized for a pre-set time;
- Emptying of the sterilization chamber (3) from the water steam/condense;
- Condensing the water steam extracted from the sterilization chamber at the preceding step and feeding the water steam condensed into water optionally together with the condense extracted from the sterilization chamber to the tank (6) for the used water;
- Drying and pressure balancing;
- Manual removal by a human operator of the sterilized material from the sterilization chamber (3);
said emptying step of the sterilization chamber (3) occurs through suctioning performed by said ejector (13) thanks to the generation of steam starting from water contained in the used water tank (6) and wherein
said ejector (13) is actuated by steam generated from water withdrawn from said second tank (6) for collecting used water,
**characterized in that**
the hydraulic circuit comprises a first a tubing (43) and a second tubing (42) connecting respectively the said first tank (5) and the said second tank (6) to the said steam generator (7) through a solenoid valve (EV2-3/2-NC) allowing to select the demineralized water tank 5 or the used water tank 6 from which water to be conveyed to the circuit can be withdrawn and a third tubing (45) for channelling the steam to a further valve (EV4-3/2-NC) that according to its actuation state sends it alternatively to the sterilization chamber (3) through a fourth tubing (46) or to the ejector (13) through a fifth tubing (47).

8. Method for performing sterilization cycles using the autoclave (1) according to claim 7, wherein the steam used to actuate said ejector (13) is condensed and the water so generated is conveyed to the used water tank (6).

## Patentansprüche

1. Hydraulischer Kreislauf für einen Wasserdampfautoklaven (1) zum Sterilisieren von Instrumenten, umfassend
in der Reihenfolge
einen ersten Behälter (5) für entmineralisiertes Wasser, das zur Erzeugung von Dampf für eine Sterilisationskammer bestimmt ist, und einen zweiten Behälter (6) zum Sammeln des verbrauchten Wassers,
eine Pumpe (15), zur Förderung des Wassers in der Reihenfolge zu den folgenden Einheiten
- einen Dampferzeuger (7),
- eine Sterilisationskammer (3), in der Dampf und zu sterilisierende Instrumente in Kontakt kommen,
- einen Dampfkondensator (12),
- einen Verteiler (16),
wobei diese Einheiten durch geeignete Leitungen (20-29 oder 40-50) und Magnetventile miteinander verbunden sind,
wobei der hydraulische Kreislauf weiterhin einen Ejektor (13) zum Absaugen des Inhalts der besagten Sterilisationskammer (3) umfasst und wobei der Ejektor (13) durch Dampf betätigt wird, der aus Wasser erzeugt wird, das aus dem zweiten Behälter (6) entnommen wurde, um gebrauchtes Wasser zu sammeln, **dadurch gekennzeichnet, dass** der hydraulische Kreislauf eine erste Leitung (43) und eine zweite Leitung (42) umfasst, die jeweils den ersten Behälter (5) und den zweiten Behälter (6) mit dem Dampferzeuger (7) über ein Magnetventil (EV2-3/2-NC) verbinden, wobei die Auswahl zwischen dem Behälter für entmineralisiertes Wasser (5) und dem Behälter für verbrauchtes Wasser (6) ermöglicht wird, aus dem das dem Kreislauf zuzuführendes Wasser entnommen werden kann, sowie eine dritte Leitung (45) zur Leitung des Dampfes zu einem weiteren Ventil (EV 4-3/2-NC), das je nach seinem Schaltzustand den Dampf wahlweise über eine vierte Leitung (46) zur Sterilisationskammer (3) oder über eine fünfte Leitung (47) zum Ejektor (13) leitet.

2. Hydraulischer Kreislauf für einen Wasserdampfautoklaven (1) zum Sterilisieren von Instrumenten nach Anspruch 1, wobei der Ejektor (13) stromabwärts des Verteilers (16) und stromaufwärts des Dampfkondensators (12) angeordnet ist.

3. Hydraulischer Kreislauf für einen Wasserdampfautoklaven (1) zum Sterilisieren von Instrumenten nach Anspruch 1 oder 2, wobei der Ejektor (13) stromabwärts des Dampfkondensators (12) und stromaufwärts des Brauchwasserbehälter (6) angeordnet ist.

4. Hydraulischer Kreislauf für einen Wasserdampfautoklaven (1) zum Sterilisieren von Instrumenten nach Anspruch 2 oder 3, wobei das aus dem Ejektor (13) austretende Wasser/Kondensat durch eine Leitung (49 und 50) in den Brauchwasserbehälter (6) geleitet wird dabei optional durch den Kondensator (12) durchströmen kann.

5. Hydraulischer Kreislauf für einen Wasserdampfautoklaven (1) zum Sterilisieren von Instrumenten nach einem oder mehreren der vorhergehenden Ansprüche, umfassend ferner ein Sicherheitsventil (8), einen Druckwandler (9), einen Druckschalter (10), einen bakteriologischen Luftfilter (11).

6. Hydraulischer Kreislauf für einen Wasserdampfautoklaven (1) zum Sterilisieren von Instrumenten gemäß einem oder mehreren der vorhergehenden Ansprüche, wobei der Ejektor aus einem geeigneten technischen Kunststoff, vorzugsweise PEEK, besteht.

7. Verfahren zur Durchführung von Sterilisationszyklen unter Verwendung eines den hydraulischen Kreislauf umfassenden Autoklaven (1) nach einem der Ansprüche 1 bis 6, das die folgenden Schritte umfasst:
- Manuelles Laden durch einen menschlichen Bediener der Sterilisationskammer (3) des Autoklaven (1) mit dem zu sterilisierenden Material, das in geeigneter Weise in Sterilisationsbeuteln/Kassetten angeordnet ist;
- Vorheizen der Sterilisationskammer (3) und der Ladung innerhalb der Sterilisationskammer;
- Entleeren der Sterilisationskammer (3) zur Entfernung der darin befindlichen atmosphärischen Luft; diese Entleerung kann in mehreren aufeinanderfolgenden Druckaufbau- und Entleerungsschritten erfolgen;
- Druckbeaufschlagen der Sterilisationskammer (3), wobei die Sterilisationskammer mit Wasserdampf mit hoher Temperatur und hohem Druck gefüllt wird;
- das eigentliche Sterilisieren durch Kontakt zwischen dem Wasserdampf mit hoher Temperatur und hohem Druck und das zu sterilisierende Material für eine vorbestimmte Zeit;
- Entleeren der Sterilisationskammer (3) vom Wasserdampf/Kondensat;
- Kondensieren des aus der Sterilisationskammer im vorhergehenden Schritt entnommenen Wasserdampfs und Zuführen des zu Wasser kondensierten Wasserdampfs, gegebenenfalls zusammen mit dem aus der Sterilisationskammer entnommenen Kondensat, in den Behälter (6) für verbrauchtes Wasser;
- Trocknen und Ausgleichen des Drucks;
- Manuelles Entnehmen des sterilisierten Materials aus der Sterilisationskammer (3) durch einen menschlichen Bediener;
der besagte Entleerungsschritt der Sterilisationskammer (3) erfolgt durch Ansaugen mittels des Ejektors (13) dank der Dampferzeugung, ausgehend von dem im Gebrauchswasserbehälter (6) enthaltenen Wasser, und wobei der Ejektor (13) durch Dampf betätigt wird, der von dem genannten zweiten Tank (6) zur Sammlung von Gebrauchswasser entnommenem Wasser erzeugt wird,
**dadurch gekennzeichnet, dass**
der hydraulische Kreislauf eine erste Leitung (43) und eine zweite Leitung (42) umfasst, die jeweils den ersten Behälter (5) und den zweiten Behälter (6) über ein Magnetventil (EV2-3/2-NC) mit dem Dampferzeuger (7) verbinden, wobei die Auswahl zwischen dem Tank für entmineralisiertes Wasser (5) und dem Tank für verbrauchtes Wasser (6), aus dem das dem Kreislauf zuzuführende Wasser entnommen werden kann ermöglicht wird, sowie eine dritte Leitung (45) vorgesehen ist zur Leitung des Dampfes zu einem weiteren Ventil (EV 4-3/2-NC), das je nach Schaltzustand den Dampf wahlweise über eine vierte Leitung (46) zur Sterilisationskammer (3) oder über eine fünfte Leitung (4 7) zum Ejektor (13) leitet.

8. Verfahren zur Durchführung von Sterilisationszyklen unter Verwendung des Autoklaven (1) gemäß Anspruch 7, wobei der zur Betätigung des Ejektors (13) verwendete Dampf kondensiert wird und das so erzeugte Wasser in den Gebrauchswasserbehälter (6) geleitet wird.

## Revendications

1. Circuit hydraulique pour autoclave à vapeur d'eau (1) pour la stérilisation d'instruments, comprenant dans l'ordre
- un premier réservoir (5) pour l'eau déminéralisée destinée à générer de la vapeur pour une chambre de stérilisation et un second réservoir (6) pour collecter l'eau utilisée,
- une pompe (15) pour acheminer l'eau, en séquence, vers les unités suivantes
- un générateur de vapeur (7),
- une chambre de stérilisation (3) dans laquelle la vapeur et les instruments à stériliser entrent en contact,
- un condenseur de vapeur (12),
- un collecteur (16), dont ses unités sont reliées entre elles à travers des tuyaux adaptés (20-29 ou 40-50) et des électrovannes,
dans lequel le circuit hydraulique comprend en outre un éjecteur (13) pour aspirer le contenu de ladite chambre de stérilisation (3) et
dans lequel ledit éjecteur (13) est actionné par la vapeur générée par l'eau prélevée dudit second réservoir (6) pour collecter l'eau utilisée,
**caractérisé en ce que** le circuit hydraulique comprend un premier tuyau (43) et un second tuyau (42) reliant respectivement ledit premier réservoir (5) et ledit second réservoir (6) audit générateur de vapeur (7) à travers une électrovanne (EV2-3/2-NC) permettant de sélectionner le réservoir d'eau déminéralisée 5 ou le réservoir d'eau utilisée 6 duquel l'eau à acheminer vers le circuit peut être prélevée et un troisième tuyau (45) pour canaliser la vapeur vers une autre vanne (EV4-3/2-NC) qui, en fonction de son état d'actionnement, l'envoie alternativement à la chambre de stérilisation (3) à travers un quatrième tuyau (46) ou à l'éjecteur (13) à travers un cinquième tuyau (47).

2. Circuit hydraulique pour autoclave à vapeur d'eau (1) pour la stérilisation d'instruments selon la revendication 1, dans lequel ledit éjecteur (13) est placé en aval dudit collecteur (16) et en amont dudit condenseur de vapeur (12).

3. Circuit hydraulique pour autoclave à vapeur d'eau (1) pour la stérilisation d'instruments selon la revendication 1 ou 2, dans lequel ledit éjecteur (13) est placé en aval dudit condenseur de vapeur (12) et en amont dudit réservoir d'eau utilisée (6).

4. Circuit hydraulique pour autoclave à vapeur d'eau (1) pour la stérilisation d'instruments selon la revendication 2 ou 3, dans lequel l'eau/le condensat sortant dudit éjecteur (13) est acheminée vers le réservoir d'eau utilisée (6) à travers un tuyau (49 et 50) et passant éventuellement à travers ledit condenser (12).

5. Circuit hydraulique pour autoclave à vapeur d'eau (1) pour la stérilisation d'instruments selon une ou plusieurs des revendications précédentes, comprenant en outre une soupape de sécurité (8), un transducteur de pression (9), un pressostat (10), un filtre à air bactériologique (11).

6. Circuit hydraulique pour autoclave à vapeur d'eau (1) pour la stérilisation d'instruments selon une ou plusieurs des revendications précédentes, dans lequel ledit éjecteur est réalisé d'un matériau plastique technique approprié, de préférence PEEK.

7. Méthode pour réaliser des cycles de stérilisation en utilisant un autoclave (1) comprenant le circuit hydraulique selon les revendications 1 à 6, comprenant les étapes suivantes:
- Charger manuellement, par un opérateur humain, la chambre de stérilisation (3) dudit autoclave (1) avec le matériau à stériliser, placé de manière appropriée à l'intérieur de sachets/cassettes de stérilisation;
- Préchauffer la chambre de stérilisation (3) et la charge à l'intérieur de ladite chambre de stérilisation;
- Vider la chambre de stérilisation (3) pour éliminer l'air à pression atmosphérique qui s'y trouve; ladite vidange peut éventuellement s'effectuer par des étapes successives de pressurisation et de vidange;
- Pressuriser la chambre de stérilisation (3), dans lequel la chambre de stérilisation est remplie de vapeur d'eau à haute température et à haute pression;
- Stérilisation proprement dite, par le contact de la vapeur à haute température et à haute pression avec le matériau à stériliser pendant une durée prédéfinie;
- Vider la chambre de stérilisation (3) de la vapeur d'eau/condensat;
- Condenser la vapeur d'eau extraite de la chambre de stérilisation dans l'étape précédente et introduire la vapeur d'eau condensée en eau, éventuellement avec le condensat extrait de la chambre de stérilisation, dans le réservoir (6) pour l'eau utilisée;
- Sécher et équilibrer la pression;
- Retirer manuellement, par un opérateur humain, le matériau stérilisé de la chambre de stérilisation (3);
ladite étape de vider la chambre de stérilisation (3) s'effectue par aspiration réalisée par ledit éjecteur (13) grâce à la génération de vapeur provenant de l'eau contenue dans le réservoir d'eau utilisée (6) et ou
ledit éjecteur (13) est actionné par la vapeur générée par l'eau prélevée dudit second réservoir (6) pour collecter l'eau utilisée,
**caractérisé en ce que**
le circuit hydraulique comprend un premier tuyau (43) et un second tuyau (42) reliant respectivement ledit premier réservoir (5) et ledit second réservoir (6) audit générateur de vapeur (7) à travers une électrovanne (EV2-3/2-NC) permettant de sélectionner le réservoir d'eau déminéralisée 5 ou le réservoir d'eau utilisée 6 duquel l'eau à acheminer vers le circuit peut être prélevée et un troisième tuyau (45) pour canaliser la vapeur vers une autre vanne (EV4-3/2-NC) qui, en fonction de son état d'actionnement, l'envoie alternativement à la chambre de stérilisation (3) à travers un quatrième tuyau (46) ou à l'éjecteur (13) à travers un cinquième tuyau (47).

8. Méthode pour réaliser des cycles de stérilisation en utilisant l'autoclave (1) selon la revendication 7, dans laquelle la vapeur utilisée pour actionner ledit éjecteur (13) est condensée et l'eau ainsi générée est acheminée vers le réservoir d'eau utilisée (6).
